# FASCICULE DE BREVET EUROPEEN

(11) **EP 0 580 503 B1**
(45) Date de publication et mention de la délivrance du brevet: **14.06.1995**
(21) Numéro de dépôt: 93401876.3
(22) Date de dépôt: 21.07.1993
(51) Int. Cl.: C07D 405/06, C07D 405/14, C07D 405/12, A61K 31/54, A61K 31/495

(54) **Aminoalkylchromones, leurs procédés de préparation et les compositions qui les contiennent**
Aminoalkylchromone, Verfahren zu ihrer Herstellung und diese enthaltende Zusammensetzungen
Aminoalkylchromones, process for their preparation and compositions containing them

(30) Priorité: 21.07.1992 FR 9208950
(43) Date de publication de la demande: 26.01.1994
(60) Demande divisionnaire: 94202873.9
(73) Titulaire: ADIR ET COMPAGNIE, 92415 Courbevoie Cédex (FR)
(72) Inventeur: Payard, Marc, F-31130 Balma (FR); Baziard-Mouysset, Geneviève, F-31000 Toulouse (FR); De Saqui-Sannes, Gilbert, F-31000 Toulouse (FR); Guardiola, Béatrice, F-92200 Neuilly sur Seine (FR); Caignard, Daniel-Henri, F-75015 Paris (FR); Renard, Pierre, F-78000 Versailles (FR); Adam, Gérard, F-78600 Le Mesnil le Roi (FR)

(56) Documents cités:
- EP-A- 0 252 005
- EP-A- 0 364 274
- EP-A- 0 428 437
- EP-A- 0 457 686
- WO-A-91/05783
- US-A- 3 767 679
- CHEMICAL ABSTRACTS, vol. 115, no. 7, 19 Août 1991, Columbus, Ohio, US; abstract no. 71529h, M.-C. SACQUET ET AL.
- CHEMICAL ABSTRACTS, vol. 109, no. 11, 12 Septembre 1988, Columbus, Ohio, US; abstract no. 92718k, G. MOUYSSET ET AL.
- CHEMICAL ABSTRACTS, vol. 106, no. 11, 16 Mars 1987, Columbus, Ohio, US; abstract no. 78636p, M. PAYARD ET AL.
- CHEMICAL ABSTRACTS, vol. 67, no. 1, 3 Juillet 1967, Columbus, Ohio, US; abstract no. 2990e, G. GRENIER ET AL.
- CHEMICAL ABSTRACTS, vol. 90, no. 19, 7 Mai 1979, Columbus, Ohio, US; abstract no. 145542w, P. DA RE ET AL.
- CHEMICAL ABSTRACTS, Vol 107, No 21, 23.11.1987 abstract number 198052n
- CHEMICAL ABSTRACTS, Vol 111, No 3, 17.07.1989 abstract number 17984g

## Description

La présente invention concerne de nouvelles aminoalkylchromones, leurs procédés de préparation et les compositions pharmaceutiques qui les contiennent.

Certaines aminoalkylchromones sont déjà connues (brevet US 3,767,679) pour leur activité dans la prévention des manifestations allergiques.

L'art antérieur le plus proche, Hommage Professeur René Truhaut, (1984), 858-863, décrit des analogues de la béfuraline ainsi que leur activité dans le traitement de la dépression.

La demanderesse a présentement découvert de nouvelles aminoalkylchromones qui possèdent de remarquables propriétés pharmacologiques.

Les composés de l'invention se révèlent notamment être de puissants ligands des récepteurs sigma (σ) et à ce titre peuvent être utilisés dans les troubles du système nerveux central, ce qui les distingue totalement des composés de l'art antérieur.

Plus particulièrement, la présente invention concerne les composés de formule (I) : 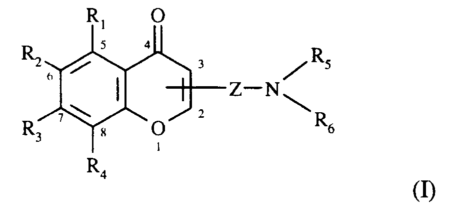
dans laquelle :
- Z: représente un chaînon méthylène éventuellement substitué par un radical acyle linéaire ou ramifié et comportant de 1 à 6 atomes de carbone,
- R₁, R₂, R₃, R₄: représentent, indépendamment les uns des autres, un radical choisi parmi :
- hydrogène,
- halogène,
- hydroxy,
- alkoxy linéaire ou ramifié et comportant de 1 à 6 atomes de carbone,
- alkyle linéaire ou ramifié et comportant de 1 à 6 atomes de carbone,
- phényle,
- benzyle,
- trifluorométhyle,
- R₅ et R₆: forment ensemble, avec l'atome d'azote qui les porte un hétérocycle choisi parmi :
- pipérazine, non substituée ou substituée,
- homopipérazine, non substituée ou substituée,
- thiomorpholine,
- azaspirane de 8 à 12 atomes, non substitué ou substitué,
- et azacycloalkyle, non substitué ou substitué,
étant entendu que, sauf précisions contraires :
- le terme "azacycloalkyle, non substitué ou substitué" désigne un squelette hydrocarboné mono- ou bi-cyclique de 8 à 15 atomes, incluant un atome d'azote et éventuellement un ou deux hétéroatomes supplémentaires choisis parmi oxygène, azote, et soufre, cet azacycloalkyle étant non substitué ou substitué par un ou plusieurs radicaux choisis parmi :
   - halogène,
   - hydroxy,
   - amino,
   - oxo,
   - mercapto,
   - alkyle linéaire ou ramifié de 1 à 4 atomes de carbone,
   - alkoxy linéaire ou ramifié de 1 à 4 atomes de carbone,
- le terme "substitué" associé aux radicaux pipérazine, homopipérazine et azaspirane signifie que ces radicaux sont substitués par un ou plusieurs radicaux choisis parmi :

- oxo,
- -R₉, 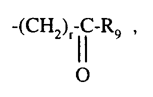 où r est égal à 0 ou à un nombre entier compris entre 1 et 4,
   et R₉ représente un radical :
- diphénylalkyle linéaire ou ramifié de 1 à 6 atomes de carbone, non substitué ou substitué sur au moins un des noyaux phényles par un ou plusieurs radicaux choisis parmi halogène, hydroxy, alkyle linéaire ou ramifié de 1 à 4 atomes de carbone, alkoxy linéaire ou ramifié de 1 à 4 atomes de carbone, et trifluorométhyle,
- (dicyclopropyl)alkyle linéaire ou ramifié de 1 à 6 atomes de carbone, non substitué ou substitué sur au moins un des cycles par un ou plusieurs radicaux choisis parmi halogène, hydroxy, alkyle linéaire ou ramifié de 1 à 4 atomes de carbone, et alkoxy linéaire ou ramifié de 1 à 4 atomes de carbone,
   - -(CH₂)ₚ-phényle, dans lequel p est égal à un nombre entier compris inclusivement entre 1 et 5, substitué ou non sur le noyau phényle par 1, 2, 3 ou 4 radicaux choisis parmi :
      - halogène,
      - hydroxy,
      - mercapto,
      - et R₁₂, non substitué ou substitué par un ou plusieurs radicaux choisis parmi halogène et hydroxy, R₁₂ représentant un radical alkyle linéaire ou ramifié de 1 à 6 atomes de carbones, ou alkoxy linéaire ou ramifié de 1 à 6 atomes de carbone,
   - -(CH₂)_{q}-naphtyle, dans lequel q est égal à 0 ou à un nombre entier compris inclusivement entre 1 et 5, substitué ou non par un ou plusieurs radicaux choisis parmi :
      - halogène,
      - hydroxy,
      - mercapto,
      - et R₁₂, non substitué ou substitué par un ou plusieurs radicaux choisis parmi halogène et hydroxy, R₁₂ ayant la même signification que précédemment,
   - R₁₀, non substitué ou substitué par un ou plusieurs radicaux halogène ou hydroxy, R₁₀ représentant un radical alkyle , alkoxy ou alkényle chacun linéaire ou ramifié et comprenant de 2 à 8 atomes de carbone
   - -(CH₂)ₜ-R₁₁, où t est égal à 0 ou à un nombre entier compris inclusivement entre 1 et 5, et R₁₁ représente un radical choisi parmi : pyrazinyle,pyrimidinyle, thiényle, pyrrolyle, furyle, thiazolyle, benzothiazolyle, pyridyle, quinolyle, isoquinolyle, benzofuryle, benzothiényle, indolyle, cycloalkyle de 3 à 8 chaînons éventuellement fusionné à un cycle benzénique, hétérocycloalkyle de 5 à 10 chaînons incluant dans son squelette carboné un hétéroatome choisi parmi oxygène, azote et soufre et éventuellement fusionné à un cycle benzénique, (1,3-benzodioxol-5-yl)alkyle linéaire ou ramifié de 1 à 6 atomes de carbone, et (1,4-benzoxan-6-yl) alkyle linéaire ou ramifié de 1 à 6 atomes de carbone,
avec la restriction que lorsque R₅ et R₆ forment ensemble, avec l'atome d'azote qui les porte, une pipérazine substituée par un groupement R₁₀, alors R₁₀ ne peut représenter un radical méthyle,
leurs isomères optiques, ainsi que leurs sels d'addition à un acide ou à une base pharmaceutiquement acceptable.

Parmi les acides utilisés pour la formation des sels d'addition des composés de formule (I), on peut citer, à titre d'exemples et de façon non limitative, dans la série minérale, les acides chlorhydrique, bromhydrique, sulfurique et phosphorique, et dans la série organique, les acides acétique, propionique, maléique, fumarique, tartrique, citrique, oxalique, benzoïque et méthanesulfonique.

Parmi les bases utilisées pour la formation des sels d'addition des composés de formule (I), on peut citer, à titre d'exemples et de façon non limitative, dans la série minérale, les hydroxydes de sodium, de potassium, de calcium et d'aluminium, les carbonates de métaux alcalinoterreux, et dans la série organique, la triéthylamine, benzylamine, diéthylamine, tertiobutylamine, dicyclohexylamine et l'arginine.

La présente invention s'étend également au procédé de préparation des composés de formule (I), caractérisé en ce que l'on fait réagir une amine de formule (II) : 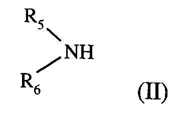
dans laquelle R₅ et R₆ sont tels que définis dans la formule (I),
avec un dérivé de l'halogénoalkyle chromone de formule (III) : 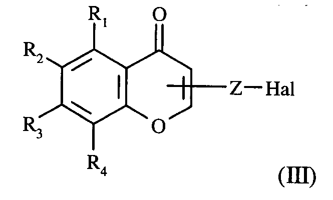
dans laquelle R₁, R₂, R₃, R₄ et Z sont tels que définis dans la formule (I) et Hal représente un atome d'halogène, afin d'obtenir les composés de formule (I),
composés de formule (I) qui peuvent être, si on le désire,
- purifiés suivant une ou plusieurs méthodes de purification choisies parmi la cristallisation, la chromatographie sur gel de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

La présente invention s'étend également au procédé de préparation des composés de formule (I/A) : 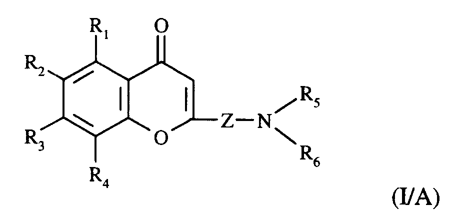
dans laquelle R₁, R₂, R₃, R₄, R₅, R₆ et Z sont tels que définis dans la formule (I),
cas particulier des composés de formule (I) pour lesquels la chromone est substituée en position 2 par le radical 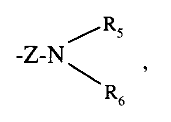
caractérisé en ce que l'on fait réagir une amine de formule (II) : 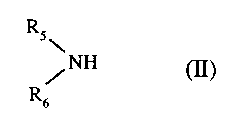
dans laquelle R₅ et R₆ sont tels que définis dans la formule (I),
avec un composé de formule (IV) :

Hal'-Z-COO-CH₂CH₃ (IV)

dans laquelle Z est tel que défini précédemment, et Hal' représente un atome d'halogène, afin d'obtenir un composé de formule (V) : 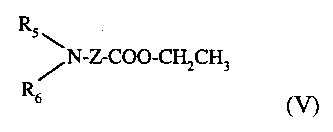
dans laquelle R₅, R₆ et Z sont tels que définis précédemment,
composé de formule (V) que l'on fait réagir avec un dérivé de l'ortho-hydroxy acétophénone de formule (VI) : 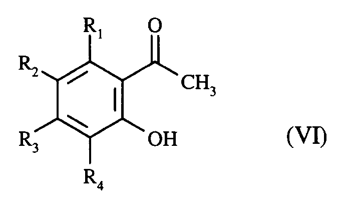
dans laquelle R₁, R₂, R₃ et R₄ sont tels que définis dans la formule (I),
afin d'obtenir les composés de formule (I/A) tels que définis précédemment, composés de formule (I/A) qui peuvent être, si on le désire,
- purifiés suivant une ou plusieurs méthodes de purification choisies parmi la cristallisation, la chromatographie sur gel de silice l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

Les matières premières utilisées dans les procédés précédemment décrits sont :
- soit commerciales,
- soit aisément accessibles à l'homme de métier selon des procédés décrits dans la littérature.

La demanderesse a découvert que les composés de l'invention possédaient des propriétés pharmacologiques très intéressantes.

Des essais de liaison aux récepteurs σ (sigma) ont en effet montré que les composés de l'invention se comportent comme de très puissants ligands de ces récepteurs ("Mesure de l'affinité des composés de l'invention pour les récepteurs σ (sigma)" : exemple B de la présente demande).

Par ailleurs, la demanderesse a découvert que les composés de formule (I) présentent une très bonne sélectivité vis à vis des récepteurs σ (sigma), notamment par rapport aux récepteurs dopaminergiques D₂ ("Mesure de l'affinité des composés de l'invention pour les récepteurs β₁, β₂, D₁, D₂, 5HT_{1A}, 5HT_{1C}, 5HT_{1D}, 5HT₂ et 5HT₃" : exemple C de la présente demande).

Cette sélectivité leur confère de remarquables propriétés neuroleptiques, antipsychotiques et psychotogéniques pures ("Etude de l'antagonisme de l'hyperactivité induite par la d-Amphétamine sulfate chez la souris" : exemple D de la présente demande et "Etude de l'antagonisme des stéréotypes comportementaux induit chez le rat par la phencyclidine" : exemple E de la présente demande), dépourvues des effets secondaires dus à la composante extra-pyramidale couramment associée aux neuroleptiques ("Etude de la réversion des effets de l'apomorphine chez le rat" : exemple F de la présente demande).

Les composés de la présente invention peuvent ainsi être utilisés dans le traitement et la prévention du stress, de l'anxiété, de la dépression, des psychoses et de la schizophrénie, sans avoir à redouter les habituels effets secondaires de type parkinsonien dont l'akinésie, les tremblements, la rigidité des membres, associés aux neuroleptiques de type classique.

La présente invention a également pour objet les compositions pharmaceutiques contenant un dérivé de formule générale (I), ou un de ses sels physiologiquement tolérables, en combinaison avec un ou plusieurs excipients ou véhicules pharmaceutiquement acceptables.

Parmi les compositions pharmaceutiques selon l'invention, on pourra citer, à titre d'exemples et de façon non limitative, celles qui conviennent à une administration par voie orale, nasale ou parentérale et notamment les comprimés, les dragées, les gélules, les paquets, les sachets, les granules, les pilules, les granulés, les suppositoires, les crèmes, les pommades, les aérosols, les capsules, les gels dermiques et les solutions injectables ou buvables.

La posologie varie d'un individu à l'autre, selon l'âge, le poids et le sexe du malade, la voie d'administration choisie, la nature et l'intensité de l'affection. Les doses utilisées s'échelonnent entre 0,1 et 100 mg pour un traitement, divisibles en 1 à 3 prises par 24 heures.

Les exemples suivants illustrent l'invention et ne la limitent en aucune façon.

### EXEMPLE 1 :

### 2-[(4-BENZYLPIPERAZIN-1-YL)METHYL]-4-OXO[4H]-1-BENZOPYRANE

### 1er procédé de synthèse

On dissout, dans 200 cm₃ de tétrahydrofurane, 0,02 mole de 2-chlorométhyl-4-oxo[4H]-1-benzopyrane. On ajoute ensuite 0,04 mole de 1-benzylpipérazine. L'évolution de la réaction est suivie par chromatographie sur couche mince. Lorsque tout le dérivé chloré a réagi, on évapore le solvant sous pression réduite. Le résidu est repris par de l'éther. La solution est filtrée, évaporée et chromatographiée sur colonne de gel de silice avec le 1,2-dichloroéthane comme éluant. On obtient le 2-[(4-benzylpipérazin-1-yl)methyl]-4-oxo[4H]-1-benzopyrane.
Point de fusion (base) : huile
Point de fusion (dichlorhydrate) : 238°C
Rendement (dichlorhydrate) : 79%
Caractéristiques spectrales (base) :

| | | |
|---|---|---|
| Infrarouge : | ν C-H, CH₂ : | 2815, 2879, 2913, 2937, 3026, 3061, 3083 cm⁻¹ |
| | ν C=O : | 1654 cm⁻¹ |
| | ν C=C : | 1463, 1570, 1607 cm⁻¹ |

| RMN (CDCl₃) | | | |
|---|---|---|---|
| δ ppm | | | |
| 2,63 | m | 8H | CH₂-N |
| 3,48 | s | 2H | Ar-CH₂-N |
| 3,52 | s | 2H | Ar-CH₂-N |
| 6,43 | s | 1H | H₃ |
| 7,27-7,77 | m | 8H | aromatiques |
| 8,2 | dd | 1H | H₅ |

### EXEMPLE 2 :

### 2-[(4-BENZYLPIPERAZIN-1-YL)METHYL]-4-OXO[4H]-1-BENZOPYRANE

### Second procédé de synthèse

### Stade A : 2-(4-benzylpipérazin-1-yl)acétate d'éthyle

Dans un erlen rodé de 500 cm³, on introduit 21 g (0,12 mole) de 1-benzylpipérazine, 13,4 cm³ soit 20 g (0,12 mole) de bromoacétate d'éthyle et 200 cm³ d'éthanol. Le mélange est porté 30 minutes à reflux. On ajoute alors 17 g (0,12 mole) de carbonate de potassium et le reflux est poursuivi encore pendant une heure. Après refroidissement, le mélange est filtré et le filtrat est évaporé sous pression réduite. Le résidu est repris par un mélange de 200 cm³ d'eau et 200 cm³ de chloroforme. Après extraction, la phase organique est séchée, filtrée et évaporée.
On obtient ainsi 29 g de 2-(4-benzylpipérazin-1-yl)acétate d'éthyle sous forme d'une huile brute brune.
Cette huile est purifée par distillation sous pression réduite. On obtient ainsi 22,5 g d'une huile visqueuse incolore.
Point de fusion (base) : huile
Rendement (base) : 72%
Caractéristiques spectrales (base) :

| | | |
|---|---|---|
| Infrarouge : | ν CH, CH₂ : | 2811, 2934, 2979, 3025 cm⁻¹ |
| | ν COO: | 1747 cm-1 |
| | ν C=C: | 1451, 1676 cm-1 |

| RMN (CDCl₃) | | | |
|---|---|---|---|
| δ ppm | | | |
| 1,2 | t | 3H | CH₃ |
| 2,56 | m | 8H | CH₃ |
| 3,13 | s | 2H | CH₂-CO |
| 3,46 | s | 2H | CH₂-aromatique |
| 4,13 | q | 2H | CH₂O |
| 7,2 | m | 5H | aromatique |

### Stade B : 2-[(4-benzylpipérazin-1-yl)méthyl]-4-oxo[4H]-1-benzopyrane

Dans un erlen de 500 cm³, on introduit 100 cm³ de pyridine anhydre, 13 g (0,05 mole) du composé obtenu au stade A et 7,5 g (0,055 mole) de 2-hydroxyacétophénone. On ajoute ensuite très lentement sous agitation et par petites portions 10 g (0,41 mole) d'hydrure de sodium à 60% dispersé dans l'huile minérale. Lorsque le dégagement gazeux a cessé, le mélange est porté à reflux et agité pendant 45 minutes. Après refroidissement, on filtre. Le précipité obtenu est lavé à l'éther puis ajouté par petites portions à 50 cm³ d'acide chlorhydrique concentré et refroidi extérieurement par de la glace. La réaction est violente. Lorsque l'addition est terminée, on chauffe a reflux pendant 5 minutes. Après refroidissement, on ajoute 200 cm³ d'eau et on extrait avec deux fois 100 cm³ de chloroforme. La phase aqueuse est alcalinisée jusqu'à pH 9 avec du carbonate de potassium et extraite à nouveau avec deux fois 100 cm³ de chloroforme. Cette dernière phase organique est séchée, filtrée et évaporée. L'huile obtenue est purifiée par chromatographie sur gel de silice en utilisant le méthyl chloroforme comme éluant. On récupère ainsi 6 g de 2-[(4-benzylpipérazin-1-yl)méthyl]-4-oxo[4H]-1-benzopyrane.
Point de fusion (base) : huile
Point de fusion (dichlorhydrate) : 238°C
Caractéristiques spectrales (base) :

| | | |
|---|---|---|
| Infrarouge : | ν CH, CH₂ : | 2815, 2879, 2913, 2937, 3026, 3061, 3083 cm⁻¹ |
| | ν C=O : | 1654 cm-1 |
| | ν C=C : | 1463, 1570, 1607 cm-1 |

| RMN (CDCl₃) | | | |
|---|---|---|---|
| δ ppm | | | |
| 2,63 | m | 8H | CH₂-N |
| 3,48 | s | 2H | Aromatique-CH₂-N |
| 3,52 | s | 2H | Aromatique-CH₂-N |
| 6,43 | s | 1H | H₃ |
| 7,27-7,77 | m | 8H | aromatiques |
| 8,2 | dd | 1H | H₅ |

### EXEMPLE 3 :

### 2-[(4-BENZYLPIPERAZIN-1-YL)METHYL]-5-CHLORO-4-OXO[4H]-1-BENZOPYR ANE

En procédant comme pour l'exemple 2, mais en remplaçant dans le stade B, le 2-hydroxyacétophénone par le 2-hydroxy-6-chloroacétophénone, on obtient le composé du titre.

### EXEMPLE 4 :

### 2-[1-(4-BENZYLPIPERAZIN-1-YL)-1-ACETYLMETHYL]-4-OXO[4H]-1-BENZOPY RANE

En procédant comme pour l'exemple 2, mais en remplaçant dans le stade A le bromoacétate d'éthyle par le 2-chloro-2-acétoacétate d'éthyle, on obtient le composé du titre.

### EXEMPLES 5 A 7 :

En procédant comme pour les exemples 2, 3, et 4, mais en remplaçant dans le stade A la 1-benzylpipérazine par la 1-{1-[di(cyclopropyl)]méthyl}pipérazine, on obtient respectivement les composés suivants :

### EXEMPLE 5 :

### 2-{[4-[1-DI(CYCLOPROPYL))METHYL]PIPERAZIN-1-YL}METHYL-4-OXO[4H]-1 -BENZO-PYRANE

### EXEMPLE 6 :

### 2-{[4-[1-(DI(CYCLOPROPYL))METHYL]PIPERAZIN-1-YL]METHYL}-5-CHLORO-4-OXO [4H]-1-BENZOPYRANE

### EXEMPLE 7 :

### 2-{1-[4-[1-(DI(CYCLOPROPYL))METHYL]PIPERAZIN-1-YL]-1-ACETYLMETHYL}-4-OXO[4H]-1-BENZOPYRANE

### EXEMPLE 8 :

### 2-{[4-(3,5-DIISOPROPYL-4-HYDROXYBENZYL)PIPERAZIN-1-YL]METHYL}-4-OXO[4H]-1-BENZOPYRANE

En procédant comme pour l'exemple 2, mais en remplaçant dans le stade A la 1-benzylpipérazine par la 1-(3,5-diisopropyl-4-hydroxybenzyl)pipérazine, on obtient le composé du titre.

### EXEMPLE 9 :

### 2-{[4-(2,5-DIMETHOXYBENZYL)PIPERAZIN-1-YL]METHYL}-4-OXO[4H]-1-BENZOPYRANE

En procédant comme pour l'exemple 2, mais en remplaçant dans le stade A la 1-benzylpipérazine par la 1-(2,5-diméthoxybenzyl)pipérazine, on obtient le composé du titre.

### EXEMPLE 10 :

### 2-{4-(3,4-DIMETHYLBENZYL)PIPERAZIN-1-YL]METHYL}-4-OXO[4H]-1-BENZOPYRANE

En procédant comme pour l'exemple 2, mais en remplaçant dans le stade A la 1-benzylpipérazine par la 1-(3,4-diméthylbenzyl)pipérazine, on obtient le composé du titre.

### EXEMPLES 11 A 18 :

En procédant comme pour l'exemple 2, mais en remplaçant dans le stade A la 1-benzylpipérazine par les dérivés de la 1-benzylpipérazine convenablement substitués, on obtient les composés suivants :

### EXEMPLE 11 :

### 2-{[4-(2,4-DIMETHOXYBENZYL)PIPERAZIN-1-YL]METHYL}-4-OXO[4H]-1-BENZOPYRANE

### EXEMPLE 12 :

### 2-{[4-(2,4-DITRIFLUOROMETHYLBENZYL)PIPERAZIN-1-YL]METHYL}-4-OXO[4H]-1-BENZOPYRANE

### EXEMPLE 13 :

### 2-{[4-(2,4-DICHLOROBENZYL)PIPERAZIN-1-YL]METHYL}-4-OXO[4H]-1-BENZOPYRANE

Point de fusion (base) : 93°C
Point de fusion (dichlorhydrate) : 198°C
Rendement (base) : 50%
Rendement (dichlorhydrate) : 84%

### EXEMPLE 14 :

### 2-{[4-(3,4-DICHLOROBENZYL)PIPERAZIN-1-YL]METHYL}-4-OXO[4H]-1-BENZOPYRANE

Point de fusion (base) : 136°C
Point de fusion (dichlorhydrate) : 222°C
Rendement (base) : 37%
Rendement (dichlorhydrate) : 88%

### EXEMPLE 15 :

### 2-{[4-(3,5-DI(TER-BUTYL)4-HYDROXYBENZYL)PIPERAZIN-1-YL]METHYL}-4-OXO[4H]-1-BENZOPYRANE

### EXEMPLE 16 :

### 2-{[4-(2,3,4-TRIMETHOXYBENZYL)PIPERAZIN-1-YL]METHYL}-4-OXO[4H]-1-BENZOPYRANE

Point de fusion (base) : huile
Point de fusion (dichlorhydrate) : 181°C
Rendement (base) : 54%

### EXEMPLE 17 :

### 2-({4-(2,4,6-TRIMETHOXYBENZYL)PIPERAZIN-1-YL]METHYL}-4-OXO[4H]-1-BENZOPYRANE

### EXEMPLE 18 :

### 2-{[4-(3,4,5-TRIMETHOXYBENZYL)PIPERAZIN-1-YL]METHYL}-4-OXO[4H]-1-BENZOPYRANE

### EXEMPLE 19 :

### 2-{4-(2,4-DICHLOROPHENETHYL)PIPERAZIN-1-YL)]METHYL}-4-OXO[4H]-1-BENZOPYRANE

En procédant comme pour l'exemple 2, mais en remplaçant dans le stade A la 1-benzylpipérazine par la 1-[2,4-dichloro(phénéthyl)]pipérazine, on obtient le composé du titre.

### EXEMPLE 20 :

### 2-[(PERHYDROAZEPIN-1-YL)METHYL]-4-OXO[4H]-1-BENZOPYRANE

En procédant comme pour l'exemple 2, mais en remplaçant dans le stade A la 1-benzylpipérazine par la perhydroazépine, on obtient le composé du titre.

### EXEMPLE 21 :

### 2-[(4-BENZHYDRYLPIPERAZIN-1-YL)METHYL]-4-OXO[4H]-1-BENZOPYRANE

En procédant comme pour l'exemple 2, mais en remplaçant dans le stade A la 1-benzylpipérazine par la 1-benzhydrylpipérazine, on obtient le composé du titre.

Point de fusion (base) : 68°C
Point de fusion (dichlorhydrate) : 234°C
Rendement (dichlorhydrate) : 91%

### EXEMPLE 22 :

### 2-{[4-[1-(4'-CHLOROPHENYL)-1-(PHENYL)METHYL]PIPERAZIN-1-YL] METHYL}-4-OXO[4H]-1-BENZOPYRANE

En procédant comme pour l'exemple 2, mais en remplaçant dans le stade A la 1-benzylpipérazine par le 1-{[1-(4'-chlorophényl)-1-phényl]méthyl} pipérazine, on obtient de la même façon le produit du titre.

### EXEMPLES 23 A 32 :

En procédant comme pour l'exemple 2, mais en remplaçant dans le stade A la 1-benzylpipérazine par les pipérazines convenablement substituées, on obtient les exemples suivants :

### EXEMPLE 23 :

### 2-{[4-[(1,3-BENZODIOXOL-5-YL)METHYL]PIPERAZIN-1-YL]METHYL}-4-OXO [4H]-1-BENZOPYRANE

Point de fusion (base) : 114-118°C
Point de fusion (dichlorhydrate) : 124°C
Rendement (base) : 59%

### EXEMPLE 24 :

### 2-{[4-[2-(1,3-BENZODIOXOL-5-YL)ETHYL]PIPERAZIN-1-YL]METHYL}-4-OXO [4H]-1-BENZOPYRANE

### EXEMPLE 25 :

### 2-{[4-(PYRIMIDIN-2-YL)PIPERAZIN-1-YL]METHYL}-4-OXO[4H]-1-BENZOPYRANE

Point de fusion (base) : 169°C
Point de fusion (dichlorhydrate) : 198°C

### EXEMPLE 26 :

### 2-{[4-(PYRID-2-YL)PIPERAZIN-1-YL]METHYL}-4-OXO[4H]-1-BENZOPYRANE

### EXEMPLE 27 :

### 2-{[4-[(PYRROLIDIN-1-YL)CARBONYLMETHYL]PIPERAZIN-1-YL]METHYL}-4-OXO[4H]-1-BENZOPYRANE

### EXEMPLE 28 :

### 2-[(4-ETHOXYCARBONYLPIPERAZIN-1-YL)METHYL]-4-OXO[4H]-1-BENZOPYRANE

### EXEMPLE 29 :

### 2-{[4-(PROPEN-2-YL)PIPERAZIN-1-YL]METHYL}-4-OXO[4H]-1-BENZOPYRANE

### EXEMPLE 30 :

### 2-{[4-(NAPHT-2-YL)PIPERAZIN-1-YL]METHYL}-4-OXO[4H]-1-BENZOPYRANE

### EXEMPLE 31 :

### 2-{[4-(2,4-DICHLOROPHENYLACETYLMETHYL)PIPERAZIN-1-YL]METHYL}-4-OXO[4H]-1-BENZOPYRANE

### EXEMPLE 32 :

### 2-{[4-(PYRROLYLMETHYLCARBONYLMETHYL)PIPERAZIN-1-YL]METHYL}-4 -OXO[4H]-1-BENZOPYRANE

### EXEMPLES 33 A 38 :

En procédant comme pour l'exemple 2, mais en remplaçant dans le stade A la 1-benzylpipérazine par les amines appropriées, on obtient les exemples suivants :

### EXEMPLE 33 :

### 2-(AZACYCLOOCTYLMETHYL)-4-OXO[4H]-1-BENZOPYRANE

Point de fusion (base) : huile
Point de fusion (chlorhydrate) : 157°C
Rendement (base) : 55%
Rendement (chlorhydrate) : 65%

### EXEMPLE 34 :

### 2-(THIOMORPHOLIN-4-YLMETHYL)-4-OXO[4H]-1-BENZOPYRANE

### EXEMPLE 35 :

### 2-[(7-OXO-8-AZASPIRO[5,4]DEC-8-YL)METHYL]-4-OXO[4H]-1-BENZOPYRANE

### EXEMPLE 36 :

### 2-[(2,4-DIOXO-3-AZASPIRO[5,5]UNDEC-3-YL)METHYL]-4-OXO[4H]-1-BENZOPYRANE

### EXEMPLE 37 :

### 2-[(8-AZABICYCLO[4.3.0]NON-8-YL)METHYL]-4-OXO[4H]-1-BENZOPYRANE

### EXEMPLE 38 :

### 2-[(7,9-DIOXO-8-AZASPIRO[4.5]DEC-8-YL)METHYL]4-OXO[4H]-1-BENZOPYRANE

### EXEMPLES 39 A 41 :

En procédant comme pour l'exemple 2, mais en remplaçant dans le stade A la 1-benzylpipérazine par les amines appropriées et en remplaçant dans le stade B la 2-hydroxyacétophénone par les dérivés de la 2-hydroxyacétophénone convenablement substitués, on obtient les composés suivants.

### EXEMPLE 39 :

### 2-(THIOMORPHOLIN-4-YLMETHYL)-8-METHOXY-4-OXO[4H]-1-BENZOPYRANE

### EXEMPLE 40 :

### 2-[(7,9-DIOXO-8-AZASPIRO[4.5]DEC-8-YL)METHYL]-4-OXO-7-TRIFLUOROMETHYL[4H]-1-BENZOPYRANE

### EXEMPLE 41 :

### 2-[(8-AZABICYCLO[4.3.0]NON-8-YL)METHYL]-7-CHLORO-4-OXO[4H]-1-BENZOPYRANE

### EXEMPLE 42 :

### 2-[(8-AZABICYCLO[4.3.0]NON-8-YL)-1-ACETYLMETHYL]-7-CHLORO-4-OXO [4H]-1-BENZOPYRANE

En procédant comme pour l'exemple 41, mais en remplaçant dans le stade A le bromoacétate d'éthyle par 2-chloro 2-acétoacétate d'éthyle, on obtient le composé du titre.

### EXEMPLE 43 :

### 3-[(4-BENZYLPIPEPAZIN-1-YL)METHYL]-4-OXO[4H]-1-BENZOPYRANE

On dissout dans 200 cm³ de tétrahydrofurane 0,02 mole de 3-chlorométhyl-4-oxo[4H]-1-benzofurane. On ajoute ensuite 0,04 mole de 1-benzylpipérazine. L'évolution de la réaction est suivie par chromatographie sur couche mince. Lorsque tout le dérivé chloré a réagi, on évapore le solvant sous pression réduite. Le résidu est repris par de l'éther. La solution est filtrée, évaporée et chromatographiée sur colonne de gel de silice avec le 1,2-dichloroéthane comme éluant. On obtient le 3-[(4-benzylpipérazin-1-yl)méthyl]4-oxo[4H]-1-benzopyrane.

### EXEMPLES 44 A 48 :

En procédant comme pour l'exemple 43, mais en remplaçant la 1-benzylpipérazine par les dérivés aminés appropriés, on obtient les composés suivants :

### EXEMPLE 44 :

### 3-{[4-(2,3,4-TRIMETHOXYBENZYL)PIPERAZIN-1-YL]METHYL}-4-OXO[4H]-1-BENZOPYRANE

### EXEMPLE 45 :

### 3-{[4-(3,4-DICHLOROBENZYL)PIPERAZIN-1-YL)METHYL}-4-OXO[4H]-1-BENZOPYRANE

### EXEMPLE 46 :

### 3-{4-(2,4-DICHLOROBENZYL)PIPERAZIN-1-YL)METHYL}-4-OXO[4H]-1-BENZOPYRANE

### EXEMPLE 47 :

### 3-[(4-NAPHT-2-YLPIPERAZIN-1-YL)METHYL]-4-OXO[4H]-1-BENZOPYRANE

### EXEMPLE 48 :

### 3-{[4-[(1,3-BENZODIOXOL-5-YL)METHYL]PIPERAZIN-1-YL]METHYL}-4-OXO [4H]-1-BENZOPYRANE

### EXEMPLE 49 :

### 2-[4-(4-METHOXYBENZYL)PIPERAZIN-1-YLMETHYL]-4-OXO[4H]-1-BENZOPYRANE

En procédant comme pour l'exemple 2, mais en remplaçant dans le stade A la 1-benzylpipérazine par la 1-(4-méthoxybenzyl)pipérazine, on obtient le composé du titre.

Point de fusion (base) : 101°C
Point de fusion (dichlorhydrate) : 226°C
Rendement (base) : 43%
Rendement (dichlorhydrate) : 97%

### EXEMPLE 50 :

### 2-[4-(4-FLUOROBENZYL)PIPERAZIN-1-YLMETHYL]-4-OXO[4H]-1-BENZOPYRANE

En procédant comme pour l'exemple 2, mais en remplaçant dans le stade A la 1-benzylpipérazine par la 1-(4-fluorobenzyl)pipérazine, on obtient le composé du titre.

Point de fusion (base) : 106°C
Point de fusion (dichlorhydrate) : 221°C
Rendement (base) : 57%
Rendement (dichlorhydrate) : 98%

### EXEMPLE 51 :

### 2-[(4-BENZYLPIPEPAZIN-1-YL)METHYL]-6-METHYL-4-OXO[4H]-1-BENZOPYRANE

En procédant comme pour l'exemple 2, mais en remplaçant dans le stade B la 2-hydroxyacétophénone par la 2-hydroxy-5-méthylacétophénone, on obtient le composé du titre.

### ETUDE PHARMACOLOGIQUE DES COMPOSES DE L'INVENTION

### EXEMPLE A : ETUDE DE LA TOXICITE DES COMPOSES DE L'INVENTION

### PROTOCOLE :

La toxicité aiguë a été appréciée après administration orale à des lots de 5 souris (20 ± 2 grammes) de doses croissantes (0,1 - 0,25 - 0,50 - 0,75 - 1 g/kg) des composés de l'invention. Les animaux ont été observés à intervalles réguliers au cours de la première journée et quotidiennement pendant les 2 semaines suivant le traitement.

### RESULTATS :

Il apparaît que les composés de l'invention sont atoxiques. Aucun décès n'est observé après administration d'une dose de 1 g.kg-1. On ne constate pas de troubles après administration de cette dose.

### EXEMPLE B : MESURE DE L'AFFINITE DES COMPOSES DE L'INVENTION POUR LES RECEPTEURS σ (SIGMA)

### PROTOCOLE :

L'affinité in vitro des composés de l'invention, pour les récepteurs σ, a été déterminée :
- par la mesure du déplacement du ³H-PPP ([3H]-(3-hydroxyphényl)-N-(1-propyl)pipéridine), ligand spécifique du récepteur σ, sur des préparations de cortex cérébral de cobaye,
- et par la mesure du déplacement du ³H-DTG (1,3-Di-(2-tolyl)guanidine), sur des préparations de cerveau de cochon d'Inde.

### RESULTATS :

Les composés de l'invention se révèlent être de très puissants ligands des récepteurs σ (sigma), avec des constantes d'affinité de l'ordre du nanomolaire.

### EXEMPLE C : MESURE DE L'AFFINITE DES COMPOSES DE L'INVENTION POUR LES RECEPTEURS β₁, β₂, D₁, D₂, 5HT_{1A}, 5HT_{1C}, 5HT_{1D}, 5HT₂ ET 5HT₃.

### PROTOCOLE:

L'affinité in-vitro des composés de l'invention a été déterminée :
- pour les récepteurs adrénergiques β₁, par la mesure du déplacement du dihydroalprénolol, sur des préparations de cortex frontal de rat,
- pour les récepteurs adrénergiques β₂, par la mesure du déplacement du dihydroalprénolol, sur des préparations de parenchyme pulmonaire de rat,
- pour les récepteurs dopaminergiques D₁, par la mesure du déplacement du SCH 23390, sur des préparations de striatum de rat,
- pour les récepteurs dopaminergiques D₂, par la mesure du déplacement du Raclopride, sur des préparations de striatum de rat,
- pour les récepteurs sérotoninergiques 5HT_{1A}, par la mesure du déplacement du 8-OH-DPAT (8-hydroxy-2-(di-n-propylamino)tétraline), sur des préparations d'hippocampes de rat,
- pour les récepteurs sérotoninergiques 5HT_{1C}, par la mesure du déplacement du N-méthylmésulergine, sur des préparations de cortex frontal et d'hippocampe de rat,
- pour les récepteurs sérotoninergiques 5HT_{1D}, par la mesure du déplacement de la 5-hydroxytryptamine, sur des préparations de cortex, striatum et globus pallidus de rat,
- pour les récepteurs sérotoninergiques 5HT₂, par la mesure du déplacement de l'amino-iodokétansérine, sur des préparations de cortex frontal de rat,
- pour les récepteurs sérotoninergiques 5HT₃, par la mesure du déplacement du BRL 43694, sur des préparations d'aéra postréma de rat.

### RESULTATS :

Les composés de l'invention présentent une affinité pour les récepteurs β₁, β₂, D₁, D₂, 5HT_{1A}, 5HT_{1C}, 5HT_{1D}, 5HT₂ ET 5HT₃ nettement plus faible que pour les récepteurs σ (sigma).

### EXEMPLE D : ETUDE DE L'ANTAGONISME DE L'HYPERACTIVITE INDUITE PAR LA d-AMPHETAMINE SULFATE CHEZ LA SOURIS

### PRINCIPE :

Ce test a pour but d'évaluer l'activité antagoniste éventuelle des composés de l'invention, sur l'hyperactivité induite par la d-amphétamine sulfate. Cette expérience permet de mettre en évidence l'activité neuroleptique d'un composé.

### PROTOCOLE :

Des souris mâles NMRI-CERJ sont placées, à raison de une par cage, dans des labyrinthes formés de 6 compartiments (25,5 × 20,5 × 9 cm) équipés de cellules photoélectriques.

Le nombre de passages de chaque animal, entre les cellules photoélectriques, est mesuré pendant 30 minutes.

Les produits de l'invention sont testés pour des doses s'échelonnant entre 8 et 32 mg/kg. Ils sont injectés, par voie intrapéritonéale, 1 heure avant le début de l'expérience.

La d-amphétamine sulfate (2 mg/kg) est quant à elle injectée par voie intrapéritonéale 30 minutes avant le début du test.

### RESULTATS :

- La d-amphétamine sulfate, injectée seule 30 minutes avant le début de l'expérience, provoque une augmentation de 113 % du nombre de passages entre les cellules photoélectriques.
- Dès 8 mg/kg, les composés de l'invention antagonisent, de façon importante, l'hyperactivité induite chez la souris par la d-amphétamine sulfate, ce qui met en évidence leur activité neuroleptique.

### EXEMPLE E : ETUDE DE L'ANTAGONISME DES STEREOTYPES COMPORTEMENTAUX INDUITS CHEZ LE RAT PAR LA PHENCYCLIDINE

### PRINCIPE :

La phencyclidine, qui est un ligand des récepteurs σ (sigma) induit chez le rat des stéréotypes comportementaux facilement reconnaissables (balancement de la tête, mouvements incontrôlés des pattes antérieures).
Les neuroleptiques classiques antagonisent les effets de la phencyclidine.

### PROTOCOLE :

On injecte à des rats mâles WISTAR CERJ, 8 mg/kg de phencyclidine, par voie sous-cutanée, puis on les place dans des cages transparentes (20 × 10 × 10 cm), à raison de 1 par cage, et on note l'intensité des stéréotypes observés.
Les produits de l'invention sont injectés par voie intrapéritonéale, à des doses s'échelonnant entre 8 et 32 mg/kg, 30 minutes avant la phencyclidine.

Les observations commencent 10 minutes après l'injection de phencyclidine et l'intensité des stéréotypes comportementaux est évaluée pendant 90 minutes.

### RESULTATS :

Dès 8 mg/kg, les composés de l'invention antagonisent de façon significative les stéréotypes comportementaux induits chez le rat par la phencyclidine.
Ce résultat met en évidence l'activité neuroleptique des composés de l'invention.

### EXEMPLE F : ETUDE DE LA REVERSION DES EFFETS DE L'APOMORPHINE CHEZ LE RAT

### PRINCIPE :

Les composés réversant les effets de l'apomorphine présentent une activité non négligeable, au niveau du système extrapyramidal.
Ce test permet donc de savoir si un composé neuroleptique est dépourvu, ou non, d'activité sur le système extrapyramidal.
Cette activité se traduit par des effets de type parkinsonien, tels que l'akinésie, les tremblements et la rigidité des membres.

### PROTOCOLE :

Les composés de l'invention sont administrés à des rats mâles SPRAGUE-DAWLEY, à des doses variant entre 1 et 16 mg/kg.
L'halopéridol (1 mg/kg) injectée dans les mêmes conditions est utilisée comme produit de référence.
Une solution d'apomorphine (1 mg/kg) est injectée par voie sous-cutanée, 30 minutes plus tard.
Les rats sont aussitôt placés dans des cages transparentes, à raison de 1 animal par cage, et on note l'intensité des stéréotypes observés après l'administration de l'apomorphine, pendant 30 minutes.

### RESULTATS :

Les composés de l'invention, même pour des doses de 32 mg/kg se révèlent inaptes à réverser les effets induits par l'apomorphine chez le rat, alors que l'halopéridol (1 mg/kg) fait totalement disparaître ces effets.
Ce test révèle l'absence d'activité des composés de l'invention sur le système extra-pyramidal. Les composés de l'invention sont donc dépourvus d'effets secondaires de type parkinsonien (akinésie, tremblements et rigidité des membres) de par leur inactivité sur le système extra-pyramidal.
Ils présentent donc des propriétés neuroleptiques et antipsycotiques pures dépourvues des effets secondaires dus à la composante extra-pyramidale couramment associée aux neuroleptiques classiques.

### EXEMPLE G : ETUDE DE L'ACTIVITE MOTRICE SPONTANEE (ACTIMETRIE)

### PROTOCOLE :

Les animaux (rats, souris) sont placés individuellement dans des cages de plexiglass équipées de cellules photoélectriques. Le test est effectué individuellement sur six animaux selon la méthode décrite par J. R. BOISSIER et al. (Arch. Int. Pharmacodyn., 158, (1965), 212-221). Le nombre d'interruptions, par chaque animal, du rayon de la cellule photo-électrique est enregistré sur un ordinateur par intervalles de 10 minutes pendant 1 heure. Le composé à tester est administré par voie i.p. immédiatement avant de placer les animaux dans leur cage. L'étude est effectuée sur 12 animaux par dose.

### REMARQUE :

Chez les animaux n'ayant pas été au préalable habitués à l'appareillage, une augmentation de l'activité pendant la première demi-heure suggère un effet anxiolytique, alors qu'une augmentation de l'activité pendant toute le durée du test reflète principalement une activité psychostimulante. Une diminution d'activité reflète généralement une activité sédative.

### RESULTATS :

Ce test a donné des résultats particulièrement intéressants après injection du composé de l'exemple 49. Ils sont reportés dans le tableau I ci-dessous :

**TABLEAU I**

| Test d'actimétrie | | | | |
|---|---|---|---|---|
| | **Nombre d'interruptions du rayon pour les temps de** | **DOSES (mg. kg⁻¹ i.p.)** | | |
| | | **16** | **32** | **44** |
| **EXEMPLE 49** | 0 à 30 min. | -12% NS (0,679) | -48% * (2,753) | -71% *** (4,809) |
| | 30 à 60 min. | +7% NS (0,124) | -51% NS (1,769) | +22% NS (0,600) |
| les pourcentages sont calculés par rapport au lot témoin. Le test a été effectué sur un lot de 12 souris. NS : non spécifique ; * = p<0,05 ; ** = p<0,01 ; *** = p<0,001 ; (N,NNN) = Test de Student | | | | |

### EXEMPLE H : ETUDE DE L'ANTAGONISME DES STEREOTYPIES

### PROTOCOLE :

On injecte aux animaux par voie i.p. de la d-amphétamine (8 mg.kg⁻¹ pour les souris, 4 mg.kg⁻¹ pour les rats) et on note de 1 à 4 l'intensité des stéréotypies (mouvements de tête, reniflements, léchages, rongements, mouvements répétitifs du corps). Les observations sont relevées toutes les 10 minutes jusqu'à disparition des stéréotypies. Le composé à tester est habituellement administré par voie i.p. 30 minutes avant l'injection de d-amphétamine. Le test est conduit en parallèle sur 6 animaux (Simon P. et al., J. Pharmacol., 3, 235-238 (1972)). Le composé de référence est l'halopéridol injecté à 0,5 mg.kg⁻¹ i.p..

### REMARQUE :

Les stéréotypies induites par stimulation de la dopamine sont antagonisées sélectivement par les neuroleptiques, mais sont régulées par une grande variété d'agents, tels que les antidépressants, les benzodiazépines, les antihistamines, les anticholinergiques et de nombreux bêta-bloquants.

### RESULTATS :

Des résultats très significatifs ont été obtenus après injection du composé de l'exemple 49, et sont présentés dans le tableau II :

**Antagonisme des stéréotypies**

| **Pourcentage d'antagonisme des stéréotypies** | **DOSES (mg.kg⁻¹ i.p.)** | | | **Référence Halopéridol (0,5 mg.kg⁻¹)** |
|---|---|---|---|---|
| | **8** | **16** | **32** | |
| Exemple 49 | 8% NS (0,415) | -8% NS (0,415) | 17% NS (0,674) | 100% *** (5,477) |
| Les pourcentages sont calculés par rapport au lot témoin. Le test a été effectué sur un lot de 6 souris. NS : non spécifique ; * = p<0,05 ; ** =p<0,01 ; *** = p<0,001 ; (N,NNN) = Test de Student | | | | |

### EXEMPLE I : ETUDE DE L'ACTIVITE ANTAGONISTE INDUITE CHEZ LE RAT PAR LA D-AMPHETAMINE

Ce test est basé sur la capacité qu'ont certains composés à inhiber l'hyperactivité induite chez le rat par 4 mg.kg⁻¹ d'amphétamine.
Des rats Sprague-Dawley pesant de 200 à 250 g reçoivent les composés à tester par voie i.p. 30 minutes avant l'administration de 4 mg.kg⁻¹ d'amphétamine.

L'activité locomotrice de ces rats est ensuite mesurée pendant les quatre heures qui suivent l'administration d'amphétamine.

Les composés de l'invention inhibent très significativement l'hyperactivité induite par la d-amphétamine, ce qui suggère qu'ils possèdent une activité antidopaminergique au niveau central. Les résultats sont indiqués dans le tableau III :

**TABLEAU III**

| Activité antagoniste induite par la d-amphétamine | | | | | |
|---|---|---|---|---|---|
| **Composé** | **Activité** | **DOSES (mg.kg⁻¹ i.p.)** | | | **Référence Halopéridol** (0,125 mg.kg⁻¹) |
| | | **8** | **16** | **32** | |
| Exemple 49 | % Antagoniste | 34% NS (1,930) | 58% ** (3,121) | 90% *** (5,707) | 82% *** (4,993) |
| | Effet intrinsèque (% par rapport à la référence) | +6% NS (0,313) | -5% NS (0,264) | -21% NS (1,173) | -9% (0,471) |
| Le test a été effectué sur un lot de 12 souris. NS : non spécifique ; * = p<0,05 ; ** =p<0,01 ; *** = p<0,001 ; (N,NNN) = Test de Student | | | | | |

### EXEMPLE J : COMPOSITION PHARMACEUTIQUE : COMPRIMES

### Comprimés dosés à 5 mg de 2-[(4-benzylpipérazin-1-yl)méthyl] 4-oxo [4H] 1-benzopyrane

### Formule de préparation pour 1000 comprimés :

| | |
|---|---|
| 2-[(4-benzylpipérazin-1-yl)méthyl] 4-oxo [4H] 1-benzopyrane | 5 g |
| Amidon de blé | 10 g |
| Amidon de maïs | 10 g |
| Lactose | 65 g |
| Stéarate de magnésium | 2 g |
| Silice | 1 g |
| Hydroxypropylcellulose | 2 g |

### EXEMPLE K : COMPOSITION PHARMACEUTIQUE : COMPRIMES

### Comprimés dosés à 10 mg de 2-{[4-(2,3,4-triméthoxybenzyl)pipérazin-1-yl]méthyl}-4-oxo [4H]-1-benzopyrane

### Formule de préparation pour 1000 comprimés :

| | |
|---|---|
| 2-{[4-(2,3,4-triméthoxybenzyl)pipérazin-1-yl]méthyl}-4-oxo[4H]-1-benzopyrane | 10 g |
| Amidon de blé | 10 g |
| Amidon de maïs | 10 g |
| Lactose | 65 g |
| Stéarate de magnésium | 2 g |
| Silice | 1 g |
| Hydroxypropylcellulose | 2 g |

## Revendications

1. Composés de formule (I) : 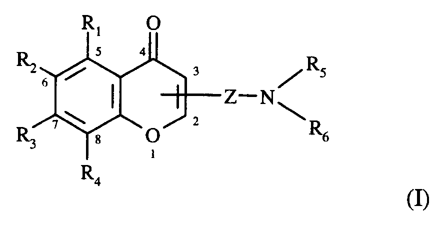 dans laquelle :
Z représente un chaînon méthylène éventuellement substitué par un radical acyle linéaire ou ramifié et comportant de 1 à 6 atomes de carbone,
R₁, R₂, R₃, R₄ représentent, indépendamment les uns des autres, un radical choisi parmi :
- hydrogène,
- halogène,
- hydroxy,
- alkoxy linéaire ou ramifié et comportant de 1 à 6 atomes de carbone,
- alkyle linéaire ou ramifié et comportant de 1 à 6 atomes de carbone,
- phényle,
- benzyle,
- trifluorométhyle,
R₅ et R₆ forment ensemble, avec l'atome d'azote qui les porte un hétérocycle choisi parmi :
- pipérazine, non substituée ou substituée,
- homopipérazine, non substituée ou substituée,
- thiomorpholine,
- azaspirane de 8 à 12 atomes, non substitué ou substitué,
- et azacycloalkyle, non substitué ou substitué,
étant entendu que, sauf précisions contraires :
- le terme "azacycloalkyle, non substitué ou substitué" désigne un squelette hydrocarboné mono- ou bi-cyclique de 8 à 15 atomes, incluant un atome d'azote et éventuellement un ou deux hétéroatomes supplémentaires choisis parmi oxygène, azote, et soufre, cet azacycloalkyle étant non substitué ou substitué par un ou plusieurs radicaux choisis parmi :
- halogène,
- hydroxy,
- amino,
- oxo,
- mercapto,
- alkyle linéaire ou ramifié de 1 à 4 atomes de carbone,
- alkoxy linéaire ou ramifié de 1 à 4 atomes de carbone,
- le terme "substitué" associé aux radicaux pipérazine, homopipérazine et azaspirane signifie que ces radicaux sont substitués par un ou plusieurs radicaux choisis parmi :
- oxo,
- -R₉, 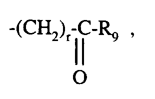 où r est égal à 0 ou à un nombre entier compris entre 1 et 4, et R₉ représente un radical :
- diphénylalkyle linéaire ou ramifié de 1 à 6 atomes de carbone, non substitué ou substitué sur au moins un des noyaux phényles par un ou plusieurs radicaux choisis parmi halogène, hydroxy, alkyle linéaire ou ramifié de 1 à 4 atomes de carbone, alkoxy linéaire ou ramifié de 1 à 4 atomes de carbone, et trifluorométhyle,
- (dicyclopropyl)alkyle linéaire ou ramifié de 1 à 6 atomes de carbone, non substitué ou substitué sur au moins un des cycles par un ou plusieurs radicaux choisis parmi halogène, hydroxy, alkyle linéaire ou ramifié de 1 à 4 atomes de carbone, et alkoxy linéaire ou ramifié de 1 à 4 atomes de carbone,
- -(CH₂)ₚ-phényle, dans lequel p est égal à un nombre entier compris inclusivement entre 1 et 5, substitué ou non sur le noyau phényle par 1, 2, 3 ou 4 radicaux choisis parmi :
- halogène,
- hydroxy,
- mercapto,
- et R₁₂, non substitué ou substitué par un ou plusieurs radicaux choisis parmi halogène et hydroxy, R₁₂ représentant un radical alkyle linéaire ou ramifié de 1 à 6 atomes de carbones, ou alkoxy linéaire ou ramifié de 1 à 6 atomes de carbone,
- -(CH₂)_{q}-naphtyle, dans lequel q est égal à 0 ou à un nombre entier compris inclusivement entre 1 et 5, substitué ou non par un ou plusieurs radicaux choisis parmi :
- halogène,
- hydroxy,
- mercapto,
- et R₁₂, non substitué ou substitué par un ou plusieurs radicaux choisis parmi halogène et hydroxy, R₁₂ ayant la même signification que précédemment,
- -R₁₀, non substitué ou substitué par un ou plusieurs radicaux halogène ou hydroxy, R₁₀ représentant un radical alkyle , alkoxy ou alkényle chacun linéaire ou ramifié et comprenant de 2 à 8 atomes de carbone
- -(CH₂)ₜ-R₁₁, où t est égal à 0 ou à un nombre entier compris inclusivement entre 1 et 5, et
R₁₁ représente un radical choisi parmi : pyrazinyle, pyrimidinyle, thiényle, pyrrolyle, furyle, thiazolyle, benzothiazolyle, pyridyle, quinolyle, isoquinolyle, benzofuryle, benzothiényle, indolyle, cycloalkyle de 3 à 8 chaînons éventuellement fusionné à un cycle benzénique, hétérocycloalkyle de 5 à 10 chaînons incluant dans son squelette carboné un hétéroatome choisi parmi oxygène, azote et soufre et éventuellement fusionné à un cycle benzénique, (1,3-benzodioxol-5-yl)alkyle linéaire ou ramifié de 1 à 6 atomes de carbone, et (1,4-benzoxan-6-yl) alkyle linéaire ou ramifié de 1 à 6 atomes de carbone,
avec la restriction que lorsuqe R₅ et R₆ forment ensemble, avec l'atome d'azote qui les porte, une pipérazine substituée par un groupement R₁₀, alors R₁₀ ne peut représenter un radical méthyle,
leurs isomères optiques, ainsi que leurs sels d'addition à un acide ou à une base pharmaceutiquement acceptable.

2. Composés de formule (I) selon la revendication 1 pour lesquels le groupement de formule 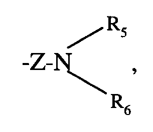 avec Z, R₅, et R₆ tels que définis dans la revendication 1, substitue en position 2 la chromone présente dans la formule (I) selon la revendication 1, leurs isomères optiques,
et leurs sels d'addition à un acide ou à une base pharmaceutiquement acceptable.

3. Composés de formule (I) selon la revendication 1 pour lesquels le groupement de formule 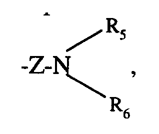 avec Z, R₅, et R₆ tels que définis dans la revendication 1, substitue en position 3 la chromone présente dans la formule (I) selon la revendication 1, leurs isomères optiques,
et leurs sels d'addition à un acide ou à une base pharmaceutiquement acceptable.

4. Composés de formule (I) selon la revendication 1 pour lesquels R₅ et R₆ forment avec l'atome d'azote qui les porte une pipérazine non substituée ou substituée, le terme "substitué" associé à la pipérazine étant tel que défini dans la revendication 1, leurs isomères optiques,
et leurs sels d'addition à un acide ou à une base pharmaceutiquement acceptable.

5. Composés de formule (I) selon la revendication 1 pour lesquels Z représente un groupement -CH₂-,
leurs isomères optiques,
et leurs sels d'addition à un acide ou à une base pharmaceutiquement acceptable.

6. Composé selon la revendication 1 qui est le 2-[(4-benzylpipérazin-1-yl)méthyl]-4-oxo[4H]-1-benzopyrane, et ses sels d'addition à un acide pharmaceutiquement acceptable.

7. Composé selon la revendication 1 qui est le 2-{[4-(2,3,4-triméthoxybenzyl)pipérazin-1-yl]méthyl}-4-oxo[4H]-1-benzopyrane, et ses sels d'addition à un acide pharmaceutiquement acceptable.

8. Composé selon la revendication 1 qui est le 2-[(4-benzhydrylpipérazin-1-yl)méthyl]-4-oxo[4H]-1-benzopyrane, et ses sels d'addition à un acide pharmaceutiquement acceptable.

9. Composé selon la revendication 1 qui est le 2-{[4-[(1,3-benzodioxyl-5-yl)méthyl]pipérazin-1-yl]méthyl}-4-oxo[4H]-1-benzopyrane, et ses sels d'addition à un acide pharmaceutiquement acceptable.

10. Composé selon la revendication 1 qui est le 2-{[4-(pyrimidin-2yl)méthyl]pipérazin-1-yl]méthyl}-4-oxo[4H]-1-benzopyrane, et ses sels d'addition à un acide pharmaceutiquement acceptable.

11. Composé selon la revendication 1 qui est le 2-(azacyclooctylmethyl)-4-oxo[4H]-1-benzopyrane, et ses sels d'addition à un acide pharmaceutiquement acceptable.

12. Composé selon la revendication 1 qui est le 2-[4-(4-méthoxybenzyl)pipérazin-1-ylméthyl]-4-oxo[4H]-1-benzopyrane et ses sels d'addition à un acide pharmaceutiquement acceptable.

13. Composé selon la revendication 1 qui est le 2-[4-(4-fluorobenzyl)pipérazin-1-ylméthyl]-4-oxo[4H]-1-benzopyrane et ses sels d'addition à un acide pharmaceutiquement acceptable.

14. Composé selon la revendication 1 qui est le 2-[(4-benzylpipérazin-1-yl)méthyl]-6-méthyl-4-oxo[4H]-1-benzopyrane et ses sels d'addition à un acide pharmaceutiquement acceptable.

15. Procédé de préparation des composés de formule (I) selon la revendication 1, caractérisé en ce que l'on fait réagir une amine de formule (II) : 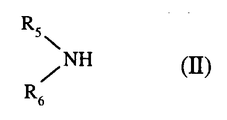 dans laquelle R₅ et R₆ sont tels que définis dans la revendication 1,
avec un dérivé de l'halogénoalkyle chromone de formule (III) : 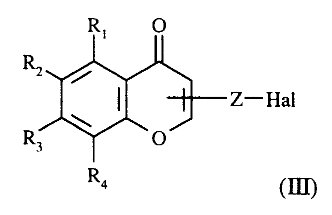 dans laquelle R₁, R₂, R₃, R₄ et Z sont tels que définis dans la revendication 1 et Hal représente un atome d'halogène, afin d'obtenir les composés de formule (I),
composés de formule (I) qui peuvent être, si on le désire,
- purifiés suivant une ou plusieurs méthodes de purification choisies parmi la cristallisation, la chromatographie sur gel de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

16. Procédé de préparation des composés de formule (I/A) : 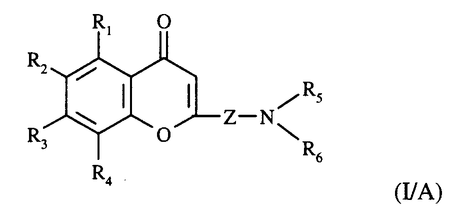 dans laquelle R₁, R₂, R₃, R4, R₅, R₆ et Z sont tels que définis dans la revendication 1, cas particulier des composés de formule (I), pour lesquels la chromone est substituée en position 2 par le radical : 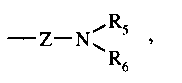 caractérisé en ce que l'on fait réagir une amine de formule (II) : 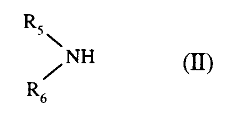 dans laquelle R₅ et R₆ sont tels que définis dans la revendication 1,
avec un composé de formule (IV) :
Hal'-Z-COO-CH₂CH₃ (IV)
dans laquelle Z est tel que défini précédemment, et Hal' représente un atome d'halogène, afin d'obtenir un composé de formule (V) : 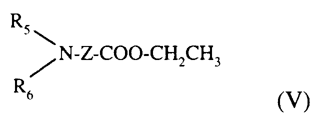 dans laquelle R₅, R₆ et Z sont tels que définis précédemment,
composé de formule (V) que l'on fait réagir avec un dérivé de l'ortho-hydroxy acétophénone de formule (VI) : 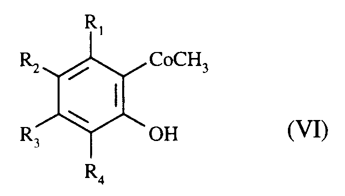 dans laquelle R₁, R₂, R₃ et R₄ sont tels que définis dans la revendication 1,
afin d'obtenir les composés de formule (I/A) tels que définis précédemment,
composés de formule (I/A) qui peuvent être, si on le désire,
- purifiés suivant une ou plusieurs méthodes de purification choisies parmi la cristallisation, la chromatographie sur gel de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

17. Composition pharmaceutique contenant comme principe actif au moins un composé selon la revendication 1, en combinaison avec un ou plusieurs excipients ou véhicules pharmaceutiquement acceptables.

18. Composition pharmaceutique selon la revendication 17 utile dans le traitement et la prévention du stress, de l'anxiété, de la dépression, des psychoses et de la schizophrénie.

19. Composition pharmaceutique selon la revendication 17 utile dans le traitement et la prévention de la dépression.

20. Composition pharmaceutique selon la revendication 17 utile dans le traitement et la prévention de la schizophrénie.

## Claims

1. Compounds of formula (I): 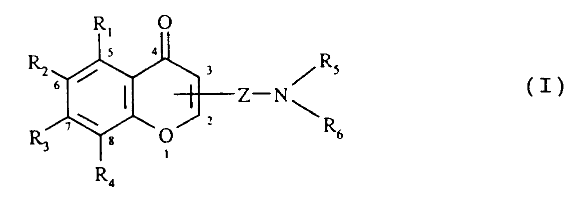 in which:
Z represents a methylene chain optionally substituted by a linear or branched acyl radical having from 1 to 6 carbon atoms,
each of R₁, R₂, R₃ and R₄, independently of the others, represents a radical selected from:
- hydrogen,
- halogen,
- hydroxy,
- linear and branched alkoxy having from 1 to 6 carbon atoms,
- linear and branched alkyl having from 1 to 6 carbon atoms,
- phenyl,
- benzyl, and
- trifluoromethyl,
R₅ and R₆ form together, with the nitrogen atom carrying them, a heterocycle selected from:
- unsubstituted and substituted piperazine,
- unsubstituted and substituted homopiperazine,
- thiomorpholine,
- unsubstituted and substituted azaspirane having from 8 to 12 atoms,
- and unsubstituted and substituted azacycloalkyl,
it being understood that, unless otherwise indicated:
- the term "unsubstituted and substituted azacycloalkyl" denotes a mono- or bi-cyclic hydrocarbon skeleton having from 8 to 15 atoms, including a nitrogen atom and optionally one or two additional hetero atoms selected from oxygen, nitrogen and sulphur, this azacycloalkyl being unsubstituted or substituted by one or more radicals selected from:
- halogen,
- hydroxy,
- amino,
- oxo,
- mercapto,
- linear and branched alkyl having from 1 to 4 carbon atoms,
- and linear and branched alkoxy having from 1 to 4 carbon atoms,
- the term "substituted" associated with piperazine, homopiperazine and azaspirane radicals denotes that those radicals are substituted by one or more radicals selected from:
- oxo,
- -R₉,
- 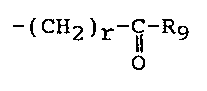 in which r is 0 or an integer from 1 to 4,
and R₉ represents
- a linear or branched diphenylalkyl radical, where alkyl has from 1 to 6 carbon atoms, which radical is unsubstituted or substituted on at least one of the phenyl rings by one or more radicals selected from halogen, hydroxy, linear and branched alkyl having from 1 to 4 carbon atoms, linear and branched alkoxy having from 1 to 4 carbon atoms, and trifluoromethyl,
- a linear or branched (dicyclopropyl)alkyl radical, where alkyl has from 1 to 6 carbon atoms, which radical is unsubstituted or substituted on at least one of the rings by one or more radicals selected from halogen, hydroxy, linear and branched alkyl having from 1 to 4 carbon atoms, and linear and branched alkoxy having from 1 to 4 carbon atoms,
- a -(CH₂)ₚ-phenyl radical in which p is an integer from 1 to 5 inclusive, which radical is unsubstituted or substituted on the phenyl ring by 1, 2, 3 or 4 radicals selected from:
- halogen,
- hydroxy,
- mercapto,
- and R₁₂ that is unsubstituted or substituted by one or more radicals selected from halogen and hydroxy, R₁₂ representing a linear or branched alkyl radical having from 1 to 6 carbon atoms or a linear or branched alkoxy radical having from 1 to 6 carbon atoms,
- a -(CH₂)_{q}-naphthyl radical in which q is 0 or an integer from 1 to 5 inclusive, which radical is unsubstituted or substituted by one or more radicals selected from:
- halogen,
- hydroxy,
- mercapto,
- and R₁₂ that is unsubstituted or substituted by one or more radicals selected from halogen and hydroxy, R₁₂ being as defined above,
- a radical -R₁₀ that is unsubstituted or substituted by one or more halogen or hydroxy radicals, R₁₀ representing an alkyl, alkoxy or alkenyl radical, each of which is linear or branched and contains from 2 to 8 carbon atoms, or
- a radical -(CH₂)ₜ-R₁₁ in which t is 0 or an integer from 1 to 5 inclusive, and R₁₁ represents a radical selected from: pyrazinyl, pyrimidinyl, thienyl, pyrrolyl, furyl, thiazolyl, benzothiazolyl, pyridyl, quinolyl, isoquinolyl, benzofuryl, benzothienyl, indolyl, cycloalkyl having from 3 to 8 ring members that is optionally fused to a benzene ring, heterocycloalkyl having from 5 to 10 ring members that includes in its carbon skeleton a hetero atom selected from oxygen, nitrogen and sulphur and is optionally fused to a benzene ring, linear or branched (1, 3-benzodioxol-5-yl)alkyl where alkyl has from 1 to 6 carbon atoms, and linear or branched (1, 4-benzoxan-6-yl)alkyl where alkyl has from 1 to 6 carbon atoms,
with the proviso that when R₅ and R₆ form together, with the nitrogen atom carrying them, a piperazine substituted by a grouping R₁₀, R₁₀ may not represent a methyl radical,
their optical isomers, and also their addition salts with a pharmaceutically acceptable acid or base.

2. Compounds of formula (I) according to claim 1 in which the grouping of the formula 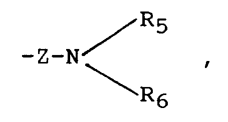 in which Z, R₅ and R₆ are as defined in claim 1, substitutes the chromone present in formula (I) according to claim 1 in the 2-position,
their optical isomers,
and their addition salts with a pharmaceutically acceptable acid or base.

3. Compounds of formula (I) according to claim 1 in which the grouping of the formula 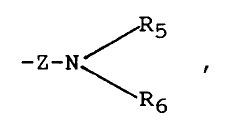 in which Z, R₅ and R₆ are as defined in claim 1, substitutes the chromone present in formula (I) according to claim 1 in the 3-position,
their optical isomers,
and their addition salts with a pharmaceutically acceptable acid or base.

4. Compounds of formula (I) according to claim 1 in which R₅ and R₆ form with the nitrogen atom carrying them an unsubstituted or substituted piperazine,
the term "substituted" associated with piperazine being as defined in claim 1,
their optical isomers,
and their addition salts with a pharmaceutically acceptable acid or base.

5. Compounds of formula (I) according to claim 1 in which Z represents a grouping -CH₂-,
their optical isomers,
and their addition salts with a pharmaceutically acceptable acid or base.

6. Compound according to claim 1 which is 2-[(4-benzylpiperazin-1-yl)methyl]-4-oxo[4H]-1-benzopyran, and its addition salts with a pharmaceutically acceptable acid.

7. Compound according to claim 1 which is 2-{[4-(2, 3, 4-trimethoxybenzyl)piperazin-1-yl]methyl}-4-oxo[4H]-1-benzopyran, and its addition salts with a pharmaceutically acceptable acid.

8. Compound according to claim 1 which is 2-[(4-benzhydrylpiperazin-1-yl)methyl]-4-oxo[4H]-1-benzopyran, and its addition salts with a pharmaceutically acceptable acid.

9. Compound according to claim 1 which is 2-{[4-[(1, 3-benzodioxyl-5-yl)methyl]piperazin-1-yl]methyl}-4-oxo[4H]-1-benzopyran, and its addition salts with a pharmaceutically acceptable acid.

10. Compound according to claim 1 which is 2-{[4-(pyrimidin-2-yl)methyl]piperazin-1-yl]methyl}-4-oxo[4H]-1-benzopyran, and its addition salts with a pharmaceutically acceptable acid.

11. Compound according to claim 1 which is 2-(azacyclooctylmethyl)-4-oxo[4H]-1-benzopyran, and its addition salts with a pharmaceutically acceptable acid.

12. Compound according to claim 1 which is 2-[4-(4-methoxybenzyl)piperazin-1-ylmethyl}-4-oxo[4H]-1-benzopyran, and its addition salts with a pharmaceutically acceptable acid.

13. Compound according to claim 1 which is 2-[4-(4-fluorobenzyl)piperazin-1-ylmethyl]-4-oxo[4H]-1-benzopyran, and its addition salts with a pharmaceutically acceptable acid.

14. Compound according to claim 1 which is 2-[(4-benzylpiperazin-1-yl)methyl]-6-methyl-4-oxo[4H]-1-benzopyran, and its addition salts with a pharmaceutically acceptable acid.

15. Process for the preparation of the compounds of formula (I) according to claim 1, characterised in that an amine of formula (II): 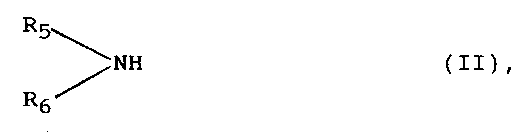 in which R₅ and R₆ are as defined in claim 1,
is reacted with a haloalkylchromone compound of formula (III): 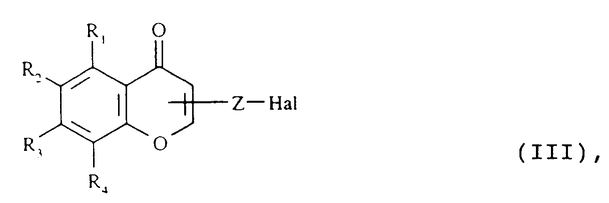 in which R₁, R₂, R₃, R₄ and Z are as defined in claim 1 and Hal represents a halogen atom,
in order to obtain the compounds of formula (I),
which compounds of formula (I) may, if desired, be
- purified in accordance with one or more purification methods selected from crystallisation, chromatography on silica gel, extraction, filtration, and passage over charcoal and/or resin,
- separated, where appropriate, in pure form or in the form of a mixture, into their possible optical isomers,
- and/or converted into a salt using a pharmaceutically acceptable acid or base.

16. Process for the preparation of the compounds of formula (I/A): 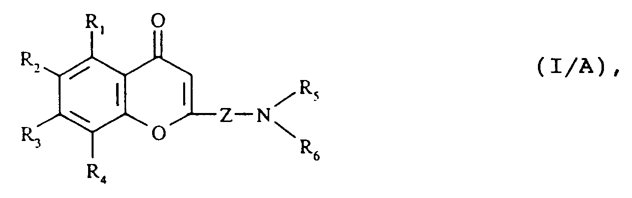 in which R₁, R₂, R₃, R₄, R₅, R₆ and Z are as defined in claim 1, which is a particular case of the compounds of formula (I) in which the chromone is substituted in the 2-position by the radical 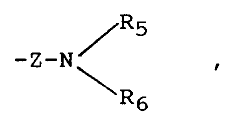 characterised in that an amine of formula (II): 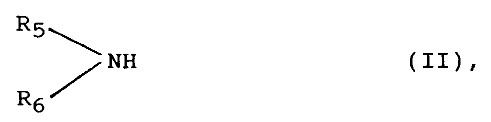 in which R₅ and R₆ are as defined in claim 1,
is reacted with a compound of formula (IV):
Hal'-Z-COO-CH₂CH₃ (IV),
in which Z is as defined above and Hal' represents a halogen atom,
in order to obtain a compound of formula (V): 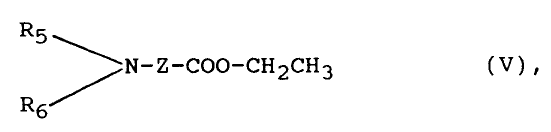 in which R₅, R₆ and Z are as defined above,
which compound of formula (V) is reacted with an ortho-hydroxyacetophenone compound of formula (VI): 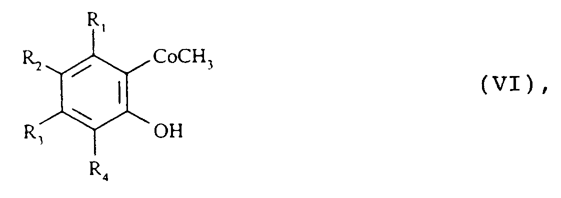 in which R₁, R₂, R₃ and R₄ are as defined in claim 1,
in order to obtain the compounds of formula (I/A) such as defined above,
which compounds of formula (I/A) may, if desired, be
- purified in accordance with one or more purification methods selected from crystallisation, chromatography on silica gel, extraction, filtration, and passage over charcoal and/or resin,
- separated, where appropriate, in pure form or in the form of a mixture, into their possible optical isomers,
- and/or converted into a salt using a pharmaceutically acceptable acid or base.

17. Pharmaceutical composition containing as active ingredient at least one compound according to claim 1, in combination with one or more pharmaceutically acceptable excipients or carriers.

18. Pharmaceutical composition according to claim 17 for use in the treatment and prevention of stress, anxiety, depression, psychoses and schizophrenia.

19. Pharmaceutical composition according to claim 17 for use in the treatment and prevention of depression.

20. Pharmaceutical composition according to claim 17 for use in the treatment and prevention of schizophrenia.

## Patentansprüche

1. Verbindungen der Formel (I): 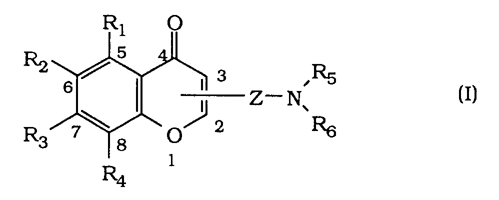 in der:
Z eine Methylenkette, die gegebenenfalls durch eine geradkettige oder verzweigte Acylgruppe mit 1 bis 6 Kohlenstoffatomen substituiert ist.
R₁, R₂, R₃, R₄ unabhängig voneinander eine Gruppe ausgewählt aus:
- Wasserstoff,
- Halogen,
- Hydroxy,
- geradkettiges oder verzweigtes Alkoxy mit 1 bis 6 Kohlenstoffatomen,
- geradkettiges oder verzweigtes Alkyl mit 1 bis 6 Kohlenstoffatomen,
- Phenyl,
- Benzyl,
- Trifluormethyl,
R₅ und R₆ gemeinsam mit dem Stickstoffatom, welches sie trägt, einen Heterocyclus ausgewählt aus:
- nichtsubstituiertes oder substituiertes Piperazin,
- nichtsubstituiertes oder substituiertes Homopiperazin,
- Thiomorpholin,
- nichtsubstituiertes oder substituiertes Azaspiran mit 8 bis 12 Atomen,
- und nichtsubstituiertes oder substituiertes Azacycloalkyl, mit der Maßgabe bedeuten, daß, wenn nichts anderes angegeben ist:
- der Begriff "nichtsubstituiertes oder substituiertes Azacycloalkyl'' ein mono- oder bicyclisches Kohlenwasserstoffskelett mit 8 bis 15 Atomen, welches ein Stickstoffatom und gegebenenfalls ein oder zwei zusätzliche Heteroatome ausgewählt aus Sauerstoff, Stickstoff und Schwefel enthält, darstellt, und dieses Azacycloalkyl nichtsubstituiert ist oder durch eine oder mehrere Gruppen ausgewählt aus:
- Halogen,
- Hydroxy,
- Amino,
- Oxo,
- Mercapto,
- geradkettiges oder verzweigtes Alkyl mit 1 bis 4 Kohlenstoffatomen,
- geradkettiges oder verzweigtes Alkoxy mit 1 bis 4 Kohlenstoffatomen
substituiert ist.
- der Begriff "substituiert", wie er für die Piperazin-, Homopiperazin- und Azaspirangruppen benutzt wird, bedeutet, daß diese Gruppen durch eine oder mehrere Gruppen substituiert sind, ausgewählt aus:
- Oxo,
- -R₉, 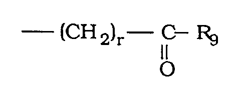 worin r 0 oder eine ganze Zahl zwischen 1 und 4 darstellt, und R₉:
- eine geradkettige oder verzweigte Diphenylalkylgruppe mit 1 bis 6 Kohlenstoffatomen, die nichtsubstituiert oder an mindestens einem der Phenylkerne durch eine oder mehrere Gruppen ausgewählt aus Halogen, Hydroxy, geradkettiges oder verzweigtes Alkyl mit 1 bis 4 Kohlenstoffatomen, geradkettiges oder verzweigtes Alkoxy mit 1 bis 4 Kohlenstoffatomen und Trifluormethyl substituiert ist,
- eine geradkettige oder verzweigte (Dicyclopropyl)-alkylgruppe mit 1 bis 6 Kohlenstoffatomen, die nichtsubstituiert oder an mindestens einem der Ringe durch eine oder mehrere Gruppen ausgewählt aus Halogen, Hydroxy, geradkettiges oder verzweigtes Alkyl mit 1 bis 4 Kohlenstoffatomen und geradkettiges oder verzweigtes Alkoxy mit 1 bis 4 Kohlenstoffatomen substituiert ist,
- eine -(CH₂)ₚ-Phenylgruppe, worin p eine ganze Zahl zwischen 1 und 5 einschließlich darstellt, die nichtsubstituiert oder am Phenylkern durch 1, 2, 3 oder 4 Gruppen ausgewählt aus:
- Halogen,
- Hydroxy,
- Mercapto,
- und R₁₂, das nichtsubstituiert oder durch eine oder mehrere Gruppen ausgewählt aus Halogen und Hydroxy substituiert ist, worin R₁₂ eine geradkettige oder verzweigte Alkylgruppe mit 1 bis 6 Kohlenstoffatomen oder eine geradkettige oder verzweigte Alkoxygruppe mit 1 bis 6 Kohlenstoffatomen darstellt, substituiert ist,
- eine -(CH₂)_{q}-Naphthylgruppe, worin q 0 oder eine ganze Zahl mit einem Wert von 1 bis 5 einschließlich darstellt, die nichtsubstituiert oder durch eine oder mehrere Gruppen ausgewählt aus:
- Halogen,
- Hydroxy,
- Mercapto,
- und R₁₂, das nichtsubstituiert oder durch eine oder mehrere Gruppen ausgewählt aus Halogen und Hydroxy substituiert ist, worin R₁₂ die oben angegebenen Bedeutungen besitzt, substituiert ist,
- R₁₀, das nichtsubstituiert oder durch eines oder mehrere Halogenatome oder Hydroxylgruppen substituiert ist, worin R₁₀ eine geradkettige oder verzweigte Alkyl-, Alkoxy- oder Alkenylgruppe mit jeweils 2 bis 8 Kohlenstoffatomen darstellt,
- -(CH₂)ₜ-R₁₁, worin t den Wert 0 oder eine ganze Zahl mit einem Wert zwischen 1 und 5 einschließlich darstellt und
R₁₁ eine Gruppe ausgewählt aus: Pyrazinyl, Pyrimidinyl, Thienyl, Pyrrolyl, Furyl, Thiazolyl, Benzothiazolyl, Pyridyl. Chinolyl, Isochinolyl, Benzofuryl, Benzothienyl, Indolyl, Cycloalkyl mit 3 bis 8 Kettengliedern, die gegebenenfalls an einen Benzolring kondensiert sind, Heterocycloalkyl mit 5 bis 10 Kettengliedern, das in seinem Kohlenstoffskelett ein Heteroatom ausgewählt aus Sauerstoff, Stickstoff und Schwefel enthält und gegebenenfalls mit einem Benzolring kondensiert ist, geradkettiges oder verzweigtes (1,3-Benzodioxol-5-yl)-alkyl mit 1 bis 6 Kohlenstoffatomen und geradkettiges oder verzweigtes (1,4-Benzoxan-6-yl)-alkyl mit 1 bis 6 Kohlenstoffatomen darstellt, bedeutet,
mit der Maßgabe, daß, wenn R₅ und R₆ gemeinsam mit dem Stickstoffatom, welches sie trägt, einen Piperazinrest darstellen, der durch eine Gruppe R₁₀ substituiert ist, R₁₀ keine Methylgruppe darstellen kann,
deren optische Isomere sowie deren Additionssalze mit einer pharmazeutisch annehmbaren Säure oder Base.

2. Verbindungen der Formel (I) nach Anspruch 1, worin die Gruppe der Formel 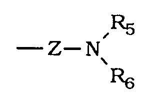 worin Z, R₅ und R₆, die in Anspruch 1 angegebenen Bedeutungen besitzen, das in der Formel (I) in Anspruch 1 angegebene Chromon in der Stellung 2 substituiert, deren optische Isomere
und deren Additionssalze mit einer pharmazeutisch annehmbaren Säure oder Base.

3. Verbindungen der Formel (I) nach Anspruch 1, worin die Gruppe der Formel 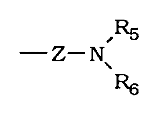 worin Z, R₅ und R₆ die in Anspruch 1 angegebenen Bedeutungen besitzen, das in der Formel (I) nach Anspruch 1 dargestellte Chromon in der Stellung 3 substituiert,
deren optische Isomere
und deren Additionssalze mit einer pharmazeutisch annehmbaren Säure oder Base.

4. Verbindungen der Formel (I) nach Anspruch 1, worin R₅ und R₆ zusammen mit dem Stickstoffatom, welches sie trägt, ein nichtsubstituiertes oder substituiertes Piperazin bilden, wobei der bezüglich des Piperazinrests benutzte Begriff "substituiert" die in Anspruch 1 angegebenen Bedeutungen besitzt,
deren optische Isomere
und deren Additionssalze mit einer pharmazeutisch annehmbaren Säure oder Base.

5. Verbindungen der Formel (I) nach Anspruch 1, worin Z eine Gruppe -CH₂- darstellt,
deren optische Isomere
und deren Additionssalze mit einer pharmazeutisch annehmbaren Säure oder Base.

6. Verbindung nach Anspruch 1, nämlich 2-[(4-Benzylpiperazin-1-yl)-methyl]-4-oxo-[4H]-1-benzopyran und dessen Additionssalze mit einer pharmazeutisch annehmbaren Säure.

7. Verbindung nach Anspruch 1, nämlich 2-{[4-(2,3,4-Trimethoxybenzyl)-piperazin-1-yl]-methyl}-4-oxo-[4H]-1-benzopyran und dessen Additionssalze mit einer pharmazeutisch annehmbaren Säure.

8. Verbindung nach Anspruch 1, nämlich 2-[(4-Benzhydrylpiperazin-1-yl)-methyl]-4-oxo-[4H]-1-benzopyran und dessen Additionssalze mit einer pharmazeutisch annehmbaren Säure.

9. Verbindung nach Anspruch 1, nämlich 2-{[4-[(1,3-Benzodioxyl-5-yl)-methyl]-piperazin-1-yl]-methyl]-4-oxo-[4H]-1-benzopyran und dessen Additionssalze mit einer pharmazeutisch annehmbaren Säure.

10. Verbindung nach Anspruch 1, nämlich 2-{[4-(Pyrimidin-2-yl)-methyl]-piperazin-1-yl]-methyl}-4-oxo-[4H]-1-benzopyran und dessen Additionssalze mit einer pharmazeutisch annehmbaren Säure.

11. Verbindung nach Anspruch 1, nämlich 2-(Azacyclooctylmethyl)-4-oxo-[4H]-1-benzopyran und dessen Additionssalze mit einer pharmazeutisch annehmbaren Säure.

12. Verbindung nach Anspruch 1, nämlich 2-[4-(4-Methoxybenzyl)-piperazin-1-yl-methyl]-4-oxo-[4H]-1-benzopyran und dessen Additionssalze mit einer pharmazeutisch annehmbaren Säure.

13. Verbindung nach Anspruch 1, nämlich 2-[4-(4-Fluorbenzyl)-piperazin-1-yl-methyl]-4-oxo-[4H]-1-benzopyran und dessen Additionssalze mit einer pharmazeutisch annehmbaren Säure.

14. Verbindung nach Anspruch 1, nämlich 2-[(4-Benzylpiperazin-1-yl)-methyl]-6-methyl-4-oxo-[4H]-1-benzopyran und dessen Additionssalze mit einer pharmazeutisch annehmbaren Säure.

15. Verfahren zur Herstellung der Verbindungen der Formel (I) nach Anspruch 1, **dadurch gekennzeichnet**, daß man ein Amin der Formel (II): 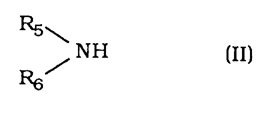 in der R₅ und R₆ die In Anspruch 1 angegebenen Bedeutungen besitzen, mit einem Halogenalkylchromon-Derivat der Formel (III): 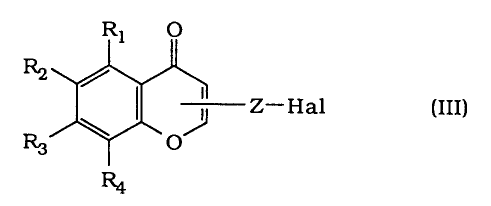 in der R₁, R₂, R₃, R₄ und Z die in Anspruch 1 angegebenen Bedeutungen besitzen und Hal ein Halogenatom darstellt,
umsetzt zur Bildung der Verbindungen der Formel (I),
welche Verbindungen der Formel (I) gewünschtenfalls
- nach einem oder mehreren Reinigungsverfahren ausgewählt aus Kristallisation, Chromatographie über Kieselgel, Extraktion, Filtration und Überführen über Aktivkohle und/oder ein Harz, gereinigt werden können,
- gegebenenfalls in reiner Form oder in Form einer Mischung ihrer gegebenenfalls vorhandenen optischen Isomeren aufgetrennt werden können,
- und/oder mit einer pharmazeutisch annehmbaren Säure oder Base in ein Salz überfuhrt werden können.

16. Verfahren zur Herstellung der Verbindungen der Formel (I/A): 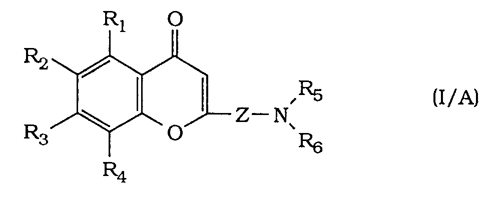 worin R₁, R₂, R₃, R₄, R₅, R₆ und Z die in Anspruch 1 angegebenen Bedeutungen besitzen, einem Sonderfall der Verbindungen der Formel (I), worin das Chromon in der 2-Stellung durch die Gruppe: 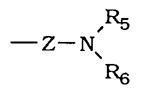 substituiert ist,
**dadurch gekennzeichnet**, daß man ein Amin der Formel (II): 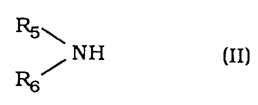 worin R₅ und R₆ die in Anspruch 1 angegebenen Bedeutungen besitzen, mit einer Verbindung der Formel (IV):
Hal' - Z - COO - CH₂CH₃ (IV)
worin Z die oben angegebenen Bedeutungen besitzt und Hal' ein Halogenatom darstellt,
umsetzt zur Bildung einer Verbindung der Formel (V): 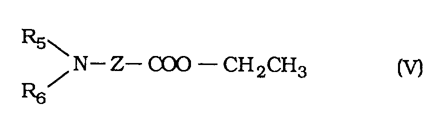 worin R₅, R₆ und Z die oben angegebenen Bedeutungen besitzen, welche Verbindung der Formel (V) man mit einem o-Hydroxy-acetophenon-Derivat der Formel (VI): 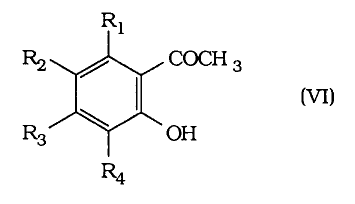 worin R₁, R₂, R₃ und R₄ die in Anspruch 1 angegebenen Bedeutungen besitzen,
umsetzt, so daß man die Verbindungen der oben definierten Formel (I/A) erhält, welche Verbindungen der Formel (I/A) gewünschtenfalls
- nach einer oder mehreren Methoden der Reinigung ausgewählt aus Kristallisation, Chromatographie über Kieselgel, Extraktion, Filtration und Überfuhren über Aktivkohle und/oder ein Harz gereinigt werden können,
- gegebenenfalls in reiner Form oder in Form einer Mischung ihrer optischen Isomeren aufgetrennt werden können,
- und/oder mit einer pharmazeutisch annehmbaren Säure oder Base in ein Salz überführt werden können.

17. Pharmazeutische Zubereitung enthaltend als Wirkstoff mindestens eine Verbindung nach Anspruch 1 in Kombination mit einem oder mehreren pharmazeutisch annehmbaren Trägermaterialien oder Bindemitteln.

18. Pharmazeutische Zubereitung nach Anspruch 17 für die Behandlung oder die Vorbeugung von Streß, der Angst, der Depression, von Psychosen und der Schizophrenie.

19. Pharmazeutische Zubereitung nach Anspruch 17 für die Behandlung und die Vorbeugung der Depression.

20. Pharmazeutische Zubereitung nach Anspruch 17 für die Behandlung und die Vorbeugung der Schizophrenie.
